# FASCICULE DE BREVET EUROPEEN

(11) **EP 3 478 649 B1**
(45) Date de publication et mention de la délivrance du brevet: **17.06.2020**
(21) Numéro de dépôt: 17732457.1
(22) Date de dépôt: 26.06.2017
(51) Int. Cl.: C07C 1/20, C07C 7/04, C07C 7/08, C07C 11/167

(54) **PROCEDE DE PRODUCTION DE BUTADIENE A PARTIR D'ETHANOL INTEGRE AVEC DISTILLATION EXTRACTIVE**
VERFAHREN ZUR HERSTELLUNG VON BUTADIEN AUS ETHANOL UNTER EINBEZIEHUNG EINER EXTRAKTIVDESTILLATION
METHOD FOR THE PRODUCTION OF BUTADIENE FROM ETHANOL, INCORPORATING EXTRACTIVE DISTILLATION

(30) Priorité: 29.06.2016 FR 1656049
(43) Date de publication de la demande: 08.05.2019
(73) Titulaire: IFP Energies nouvelles, 92852 Rueil-Malmaison (FR); Compagnie Générale des Etablissements Michelin, 63000 Clermont-Ferrand (FR)
(72) Inventeur: DASTILLUNG, Rejane, 69005 Lyon (FR); COUDERC, Sophie, 92200 Neuilly Sur Seine (FR); THINON, Olivier, 42300 Roanne (FR)
(86) Numéro de dépôt international: PCT/EP2017/065742
(87) Numéro de publication internationale: WO 2018/001982

(56) Documents cités:
- WO-A1-2016/042095
- GB-A- 829 021

## Description

### DOMAINE TECHNIQUE DE L'INVENTION

L'invention concerne un procédé de production de butadiène à partir d'éthanol.

### ART ANTÉRIEUR

Le procédé de production de 1,3-butadiène à partir d'éthanol, en une ou deux étape(s) réactionnelle(s), a un taux de conversion par passe limité. Ceci induit des recyclages importants, rendus complexes par le grand nombre d'impuretés co-produites avec le 1,3-butadiène, dont l'extraction obère le rendement global du procédé. Chaque perte dans les opérations unitaires, en particulier dans les nombreuses opérations de séparation, se traduit donc par une perte globale au sein du procédé qui devient rapidement inacceptable d'un point de vue économique. Ces pertes ont conduit à l'arrêt d'exploitation de ces procédés à l'issue de la seconde guerre mondiale.

Parmi les impuretés, on peut citer des hydrocarbures comprenant de 1 à 16 atomes de carbone, saturés ou insaturés, voire aromatiques, ainsi que des produits oxygénés tels que des alcools, des phénols, des aldéhydes, des cétones, des acides, des esters, des éthers, des acétals, saturés ou insaturés, voire aromatiques.

Dans les conditions normales de température et de pression, on peut citer parmi les sous-produits gazeux principaux l'hydrogène, le monoxyde de carbone, le dioxyde de carbone, les alcanes et oléfines en C₁-C₄ et le méthyle éthyle éther, et parmi les sous-produits liquides principaux les pentènes, les pentadiènes, le diéthyl éther, l'éthyle vinyl éther, les hexènes, les hexadiènes, le butanal, le crotonaldéhyde, l'acétate d'éthyle, le diéthyle acétal, le butanol, l'hexanol, l'acide acétique.

D'autres sous-produits sont générés en quantité infimes. Dans la suite du document, on parlera d' « huiles brunes » pour designer l'ensemble de centaines de composés hydrocarbures et oxygénés produits dans les sections réactionnelles dont les températures d'ébullition sont comprises entre celle de l'éthanol et allant jusqu'à 600°C. Ces huiles brunes ont la particularité d'être solubles dans l'éthanol, mais insolubles dans l'eau. Elles peuvent, partout où elles ne sont pas diluées par un fort excès d'éthanol, encrasser et boucher les équipements. Par ailleurs, ces huiles brunes sont problématiques au sein de la colonne à distiller qui sépare l'eau produite par la réaction et l'éthanol non converti. En effet, ces huiles brunes sont solubles dans l'effluent eau-éthanol alimentant ladite colonne à distiller, et insolubles dans le résidu essentiellement constitué d'eau. Une séparation de phase se produit donc au sein de cette colonne, diminuant considérablement l'efficacité de la séparation. Les huiles brunes sont difficiles à éliminer au sein du procédé du fait qu'elles sont constituées de centaines de composés ayant des propriétés physico-chimique très différentes. Une fraction de ces huiles brunes s'accumule donc au sein du procédé, entrainant une baisse de son efficacité au bout de quelques jours, au mieux semaines, d'opération et nécessitant de purger périodiquement certains flux. La perte en éthanol et en acétaldéhyde engendrée dégrade le rendement global du procédé pour un coût qui serait aujourd'hui rédhibitoire.

La purification du butadiène fait appel à une combinaison de nombreuses opérations unitaires, telles que des lavages, des distillations simples et extractives. L'art antérieur enseigne l'utilisation de distillations extractives mettant en oeuvre un solvant bis(2-chloroéthyle)éther, ou Chlorex, aujourd'hui proscrit car très toxique. Il est important de noter que les spécifications du butadiène sont aujourd'hui extrêmement sévères, de par la sensibilité des catalyseurs de polymérisation du butadiène. Par exemple, la spécification en acétaldéhyde (réactif intermédiaire permettant de produire le butadiène) dans le butadiène est passée de 1000 ppm à moins de 10 ppm aujourd'hui. L'ouvrage « Synthetic rubber from Alcohol», (A. Talalay, M. Magat, 1945) donne une vue générale des procédés développés jusque dans les années 40.

Le brevet US 2,409,250 décrit les étapes de purification successives du butadiène (extraction, première purification et ultime purification du butadiène par super-fractionnement). Le butadiène est produit à une pureté de 98,7%, mais au prix d'une perte significative de rendement. Pour limiter cette perte, les produits de têtes de la colonne de purification du butadiène par super-fractionnement sont soutirés et en partie recyclés vers l'étape d'extraction du butadiène. Ces recyclages importants, en particulier le recyclage du flux butène/butadiène en vue d'éliminer les incondensables, induisent un surdimensionnement des équipements.

Le brevet US 1,948,777 décrit dans le détail l'étape d'ultime purification du butadiène par distillation extractive mettant en oeuvre différents solvants, dont le Chlorex. En limitant la perte en butadiène en tête de colonne, soit une concentration de 0,2% de butadiène dans la distillat, la pureté du butadiène obtenu en fond n'est que de 70%, alors qu'en cherchant à obtenir un butadiène plus pur en fond, soit 99%, la perte en butadiène en tête est beaucoup plus importante, avec une concentration de butadiène dans le distillat de 30%. La production d'un butadiène de haute pureté est donc réalisée au prix d'une très forte baisse de rendement global de l'unité.

Le document WO14199348 décrit une méthode d'obtention du butadiène à partir d'un effluent contenant de l'éthanol et éventuellement de l'acétaldéhyde sur un catalyseur à base d'un matériau zéolitique. Des conversions d'éthanol supérieures à 95 % et des sélectivités en butadiène entre 20 et 48 % sont obtenues. Le brevet mentionne la production de composés oxygénés tel que le diéthyl éther, le crotonaldéhyde et l'éthylacétate qui sont séparés du butadiène par distillation sans qu'il soit donné plus de détails. Aucune information n'est fournie sur la gestion des nombreuses autres impuretés pouvant être présentes et sur les moyens de purification du butadiène afin de répondre aux spécifications requises quant à son utilisation dans les procédés avals.

Le catalyseur se désactive au cours de son utilisation avec pour conséquence une dégradation de la sélectivité en butadiène et la production en plus grande quantité d'impuretés telles que les butyne-1, le 1,2-butadiène, le n-butane et les butènes. Ces impuretés se retrouvent partiellement ou totalement entraînées avec le butadiène dans les étapes permettant de séparer celui-ci de l'acétaldéhyde et l'éthanol.

Les documents WO2016/042096 et WO2016/042095 décrivent un procédé de production de butadiène à partir d'une charge éthanol, respectivement en 1 et 2 étapes réactionnelles, avec un agencement d'opérations unitaires permettant d'éliminer les impuretés gazeuses et liquides tout en minimisant la perte en éthanol et acétaldéhyde, améliorant ainsi le rendement global de l'unité tout en réduisant le flux global d'eau nécessaire aux étapes de séparation et en obtenant un butadiène très pur. Dans ces procédés, la charge éthanol est utilisée pour laver l'effluent de la section réactionnelle afin de solubiliser le butadiène présent dans l'effluent vapeur qui serait sans cela en partie purgé avec les gaz légers La charge éthanol est donc mise au contact d'impuretés avant d'être alimentée dans les étapes réactionnelles. La purification finale du butadiène est réalisée par extraction liquide-liquide. Cependant, des impuretés spécifiques tel que les butynes peuvent être produites dans des teneurs critiques au regard de la spécification du produit butadiène obtenu par le procédé lorsque les catalyseurs se désactivent. Ce problème n'est pas adressé dans ces brevets. Par ailleurs, l'étape d'extraction liquide-liquide proposée nécessite une pré-purification de l'effluent butadiène pour éliminer les composés légers, l'eau résiduelle et des composés oxygénés tel que l'acétaldéhyde, l'éthanol ou le diéthyléther.

Aujourd'hui, la principale source de butadiène est pétrolière. Ce dernier est extrait d'une coupe C₄ produite par vapocraquage de naphta, contenant entre 35 et 60 % pds de butènes/butanes, entre 30 et 60 % pds de butadiène, typiquement 0.5 à 2 % pds (parfois plus) d'acétylènes, en particulier du vinylacétylène, ainsi qu'une faible quantité de 1,2-butadiène (de l'ordre de 0.1 % pds) et de composés légers comme le propane et le propylène. L'extraction est réalisée par une distillation extractive avec un solvant polaire aprotique avec une pureté supérieure à 99.5% pds et un rendement généralement supérieur à 98 % pds. Les distillations extractives utilisant la N-méthylpyrrolidone (NMP), le diméthylformamide (DMF) et l'acétonitrile (ACN) comme solvant sont les plus utilisés et les plus répandus.

La distillation extractive appliquée aux coupes C₄ issues du vapocraquage est une alternative, mais son extrapolation aux effluents butadiène issus des procédés de production à partir d'éthanol n'est pas évidente en raison de compositions différentes, à savoir une concentration en butadiène supérieure à 80 % pds et en butènes et butanes inférieure à 15% pds, une teneur en acétylènes globalement plus faible et la présence potentielle de composés oxygénés comme l'acétaldéhyde, le diéthyl éther (DEE) et l'eau, et d'alcynes produits avec la désactivation progressive des catalyseurs.

### OBJET ET INTÉRÊT DE L'INVENTION

L'invention a pour objet un procédé de production de butadiène à partir d'une charge éthanol comprenant au moins 80% poids d'éthanol, ledit procédé comprenant :
A) une étape de conversion de l'éthanol en butadiène comprenant au moins une section réactionnelle alimentée par au moins une fraction de ladite charge éthanol par au moins une fraction de l'effluent éthanol issu de l'étape E), opérée à une pression comprise entre 0,1 et 1,0 MPa et à une température comprise entre 200 et 500°C en présence d'un catalyseur, et produisant au moins un effluent réactionnel ;
B) une étape de séparation des réactifs alimentée au moins par ledit effluent réactionnel et produisant au moins un effluent éthanol-butadiène, un effluent impuretés et un effluent butadiène hydraté ;
C) une étape de séparation alimentée au moins par ledit effluent éthanol-butadiène et produisant au moins un distillat butadiène et un résidu réactifs pollués ;
D) une étape de purification du butadiène comprenant :
   - une section de séparation du butène-1 comprenant une distillation extractive, alimentée par ledit effluent butadiène hydraté issu de l'étape B), en mélange avec ledit distillat butadiène issu de l'étape C) et par un flux comprenant un solvant, et séparant en tête un effluent gaz légers, et en fond un résidu butadiène et comprenant également une distillation alimentée par ledit résidu butadiène et séparant en tête un distillat butadiène étêté et en fond un résidu solvant ;
   - une section de séparation des oxygénés comprenant une distillation extractive alimentée par ledit distillat butadiène étêté issu de la section de séparation du butène-1 et par un flux comprenant un solvant et produisant en tête un effluent butadiène pré-purifié et en fond un résidu solvant usé et comprenant également une distillation alimentée par ledit résidu solvant usé et séparant en tête un effluent composés oxygénés et en fond un résidu solvant ;
   - une section de distillation finale alimentée par ledit effluent butadiène pré-purifié issu de la section de séparation des oxygénés séparant en tête un effluent butadiène purifié et en fond un résidu butène-2 ;
E) une étape traitement des effluents alimentée au moins par le résidu réactifs pollués issu de l'étape C) et par l'effluent composés oxygénés issu de l'étape D), et produisant au moins un effluent éthanol, un effluent éthanol-acétaldéhyde, un ou plusieurs effluents huiles brunes ;

Le procédé selon l'invention permet un gain énergétique important au regard de l'art antérieur. En particulier, l'absence de traitement de la fraction vapeur de l'effluent réactionnel par lavage avec un flux d'éthanol suivi d'un lavage à l'eau permet de réduire les débits de circulation d'éthanol et d'eau dans les sections de traitement des effluents et, du fait de cette réduction, de soulager les dites sections.

Le procédé selon l'invention permet également de produire un butadiène aux spécifications, même lorsque des sous-produits, en particulier les butynes, sont générés en plus grande quantité du fait de la désactivation des catalyseurs mis en oeuvre dans les étapes réactionnelles, sans perte de rendement globale et avec un gain énergétique maintenu.

### DESCRIPTION DÉTAILLÉE DE L'INVENTION

### Charge

La charge éthanol utilisée dans le procédé selon l'invention peut provenir de toute origine, fossile, végétale ou animale, et en particulier des procédés de production d'éthanol à partir de ressources végétale. Ladite charge comprend au moins 80% poids d'éthanol, préférentiellement au moins 90% poids, et de manière préférée au moins 93 % poids. De manière très préférée, ladite charge éthanol répond aux spécifications d'éthanol carburant EN 15376.

### Étape A) de conversion de l'éthanol en butadiène

Le procédé selon l'invention comprend une étape a) de conversion de l'éthanol en butadiène.

Ladite étape A) comprend au moins une section réactionnelle alimentée par au moins une fraction de ladite charge éthanol et par au moins une fraction de l'effluent éthanol issu de l'étape E), opérée à une pression comprise entre 0,1 et 1,0 MPa et à une température comprise entre 200 et 500°C en présence d'un catalyseur, et produisant au moins un effluent réactionnel.

Dans un premier arrangement particulier du procédé selon l'invention, ladite section réactionnelle de ladite étape A) comprend une zone réactionnelle, alimentée par au moins une fraction de ladite charge éthanol et par au moins une fraction de l'effluent éthanol issu de l'étape E), permettant de convertir l'éthanol en au moins du butadiène. Elle est opérée en présence de tout catalyseur connu de l'Homme du métier, par exemple un catalyseur de type silice avec un oxyde de magnésium, à une température avantageusement comprise entre 300 et 400°C, de manière préférée entre 320 et 370°C et à une pression avantageusement comprise entre 0,1 et 0,5 MPa, de manière préférée entre 0,1 et 0,3 MPa.

Dans un deuxième arrangement particulier du procédé selon l'invention, ladite section réactionnelle de ladite étape A) comprend deux zones réactionnelles, la première, alimentée par au moins une fraction de ladite charge éthanol et par au moins une fraction de l'effluent éthanol issu de l'étape E), permettant de convertir l'éthanol en acétaldéhyde, et la seconde, alimentée par l'effluent de la première zone réactionnelle, par la fraction résiduelle dudit effluent éthanol issu de l'étape E) et par au moins une fraction de l'effluent éthanol-acétaldéhyde issu de l'étape E), permettant de convertir le mélange d'éthanol et d'acétaldéhyde en au moins du butadiène. L'effluent de la seconde zone réactionnelle constitue l'effluent réactionnel de ladite étape A).

Avantageusement, dans ce deuxième arrangement, un moyen de séparation gaz-liquide est mis en oeuvre entre les deux zones réactionnelles pour séparer l'effluent de la première section réactionnelle en un effluent gaz et un effluent liquide. L'effluent gaz, comprenant en particulier de l'hydrogène, peut être traité de la même manière que l'effluent hydrogène selon les étapes de traitement de l'hydrogène C1) et C2) décrites dans WO2016/042095. L'effluent liquide alimente la deuxième section réactionnelle.

Dans ce deuxième arrangement, ladite première zone réactionnelle est opérée en présence d'un catalyseur comprenant un oxyde de cuivre, ou de tout autre catalyseur adapté bien connu de l'Homme du métier.

Le rapport molaire éthanol sur acétaldéhyde en entrée de ladite seconde zone réactionnelle est compris entre 1 et 5, de manière préférée compris entre 1 et 3,5, de manière encore plus préférée entre 2 et 3,5 et de manière très préférée entre 2,2 et 3,5. Ladite seconde zone réactionnelle est opérée en présence d'un catalyseur, avantageusement un catalyseur supporté sur silice choisi dans le groupe constitué par les catalyseurs comprenant de l'oxyde de tantale, de zirconium ou de niobium, préférentiellement comprenant 2% d'oxyde de Tantale (voir par exemple Corson, Jones, Welling, Hincbley, Stahly, Ind. Eng Chem. 1950, 42, 2, 359-373). Ladite seconde zone réactionnelle est opérée à une température comprise entre 300 et 400°C, de manière préférée entre 320 et 370°C et à une pression comprise entre 0,1 et 1,0 MPa, de préférence entre 0,1 et 0,5 MPa, de manière préférée entre 0,1 et 0,3 MPa.

L'effluent réactionnel de ladite zone réactionnelle dans le premier arrangement particulier selon l'invention, ou de ladite seconde zone réactionnelle dans le deuxième arrangement particulier selon l'invention, comprend encore de l'éthanol, ainsi que de nombreuses impuretés produites avec le butadiène, parmi lesquelles de l'hydrogène, de l'éthylène, du propylène, du diéthyl ether (DEE), de l'acétate d'éthyle, du butanol, de l'hexanol, des butènes, des butynes, des pentènes, des pentadiènes, des hexènes, des hexadiènes, du crotonaldéhyde, du butyraldéhyde, du diéthylacétal, et de l'acide acétique. Il alimente l'étape B) de séparation des réactifs.

### Étape B) de séparation des réactifs

Le procédé selon l'invention comprend une étape B) de séparation des réactifs alimentée au moins par ledit effluent réactionnel et produisant au moins un effluent éthanol-butadiène, un effluent impuretés et un effluent butadiène hydraté.

Ladite étape B) permet de séparer d'une part un effluent liquide éthanol-butadiène comprenant majoritairement, c'est-à-dire à plus de 50% poids, les réactifs éthanol et acétaldéhyde n'ayant pas réagi, et comprenant également du butadiène, et d'autre part un effluent vapeur butadiène hydraté comprenant majoritairement, c'est-à-dire à plus de 50% poids, de butadiène.

Dans un premier arrangement préféré, ladite étape B) comprend :
- une section de séparation gaz-liquide séparant ledit effluent réactionnel en un effluent liquide constituant ledit effluent éthanol-butadiène et un effluent gaz,
- une section de compression comprimant ledit effluent gaz en un effluent gaz comprimé,
- une section de distillation alimentée par ledit effluent gaz comprimé et séparant en tête ledit effluent butadiène hydraté et en fond ledit effluent impuretés.

Ladite section de séparation gaz-liquide dudit premier arrangement met en oeuvre des moyens de séparation gaz-liquide connus de l'Homme du métier. Lesdits moyens sont opérés à une pression comprise entre 0,1 et 0,3 MPa et une température comprise entre 10 et 50°C.

Dans une première variante préférée dudit premier arrangement, ladite section de séparation gaz-liquide est opérée à une température comprise entre 10 et 20°C, préférentiellement entre 12 et 17 °C. Cette température d'opération permet de séparer dans l'effluent gaz entre 85 et 90 % du butadiène compris dans l'effluent réactionnel, ledit effluent gaz contenant alors plus de 80 % de butadiène. Le débit alimentant le compresseur est réduit par rapport à la seconde variante présentée ci-après. La teneur en éthanol, acétaldéhyde, eau et impuretés dans ledit effluent gaz est également réduite par rapport à la seconde variante présentée ci-après.

Dans une seconde variante préférée dudit premier arrangement, ladite section de séparation est opérée à une température comprise entre 40 et 50°C, préférentiellement entre 42 et 47°C. Cette température d'opération permet de séparer dans ledit effluent gaz entre 90 et 96 % du butadiène compris dans l'effluent réactionnel, ledit effluent gaz contenant alors plus de 60 % de butadiène. Le débit vers l'étape C est ainsi réduit par rapport à la première variante et la perte en butadiène limitée.

Ladite section de compression dudit premier arrangement comprime ledit effluent gaz à une pression comprise entre 0,1 et 1,0 MPa, préférentiellement entre 0,1 et 0,7 MPa et de manière préférée entre 0,2 et 0,5 MPa. L'objectif de cette section est principalement de réduire le débit volumique de gaz à traiter.

Ladite section de distillation dudit premier arrangement sépare en tête ledit effluent butadiène hydraté, lequel comprend plus de 90% poids, avantageusement plus de 95% poids, et de manière préférée plus de 98% poids, préférentiellement plus de 99% poids du butadiène compris dans ledit effluent gaz comprimé et en fond ledit effluent impuretés. Ledit effluent impuretés est avantageusement envoyé vers l'étape E) de traitement des effluents en mélange avec le résidu réactifs pollués issu de l'étape C).

Dans un deuxième arrangement préféré, ladite étape B) comprend :
- une première section de séparation gaz-liquide séparant ledit effluent réactionnel en un effluent liquide constituant ledit effluent impuretés et un effluent gaz, opérée à une température comprise entre 70 et 80°C, et une pression comprise entre 0,1 et 0,3 MPa,
- une section de compression comprimant ledit effluent gaz en un effluent gaz comprimé,
- une seconde section de séparation gaz-liquide alimentée par ledit effluent gaz comprimé et séparant en tant que phase gaz ledit effluent butadiène hydraté et en tant que phase liquide ledit effluent éthanol-butadiène, opérée à une température comprise entre 40 et 50°C et une pression comprise entre 0.2 et 0.6 MPa.

Ladite première section de séparation gaz-liquide dudit deuxième arrangement met en oeuvre des moyens de séparation gaz-liquide connus de l'Homme du métier. Lesdits moyens sont opérés à une pression comprise entre 0,1 et 0,3 MPa et une température comprise entre 70 et 80°C. L'opération de ladite section à température élevée a pour effet de minimiser la teneur de butadiène (à moins de 0,1% poids) dans ledit effluent impuretés qui alimente alors directement l'étape E) sans qu'il soit besoin de passer par l'étape C) pour récupérer le butadiène.

Ladite section de compression dudit deuxième arrangement comprime ledit effluent gaz à une pression comprise entre 0,1 et 1,0 MPa, préférentiellement entre 0,1 et 0,7 MPa et de manière préférée entre 0,2 et 0,6 MPa. L'objectif de cette section est principalement de réduire le débit volumique de gaz à traiter.

Ladite seconde section de séparation gaz-liquide dudit deuxième arrangement met en oeuvre des moyens de séparation gaz-liquide connus de l'Homme du métier. Lesdits moyens sont opérés à une pression comprise entre 0,2 et 0,6 MPa et une température comprise entre 40 et 50°C. L'opération de ladite seconde section à température plus faible a pour effet de réduire la quantité d'éthanol, d'acétaldéhyde, de diéthyléther et d'autres composés oxygénés présent dans ledit effluent butadiène hydraté envoyés vers ladite étape D).

Dans une variante préférée dudit deuxième arrangement, ledit effluent butadiène hydraté issu de ladite seconde section de séparation gaz-liquide alimente une section de distillation visant à réduire encore la quantité d'éthanol, d'acétaldéhyde et de diéthyléther présent dans ledit effluent butadiène hydraté. Cette section de distillation sépare dudit effluent butadiène hydraté en fond un résidu oxygénés et en tête un effluent butadiène hydraté appauvri en composés oxygénés, ledit effluent comprenant plus de 90% poids, avantageusement plus de 95% poids, et de manière préférée plus de 98% poids, préférentiellement plus de 99% poids du butadiène compris dans ledit effluent butadiène hydraté alimentant ladite section de distillation. Ledit effluent butadiène hydraté appauvri en composés oxygénés alimente alors l'étape D) en tant qu'effluent butadiène hydraté. Ledit résidu oxygénés alimente avantageusement ladite étape E) de traitement des effluents en mélange avec ledit résidu réactifs pollués issu de l'étape C).

Dans une autre variante préférée dudit premier ou dudit deuxième arrangement, ledit effluent butadiène hydraté obtenu à l'issu de ladite section de distillation, ou de ladite seconde section de séparation gaz-liquide, alimente une section de lavage à l'eau visant à réduire encore la quantité des composés polaires solubles dans l'eau tel que l'éthanol et l'acétaldéhyde présent dans ledit effluent butadiène hydraté. Cette section de lavage à l'eau comprend au moins une section de lavage gaz-liquide alimentée en fond par ledit effluent butadiène hydraté et en tête par un flux d'eau, avantageusement d'origine externe audit procédé de production de butadiène, ladite section de lavage produisant en tête un effluent butadiène hydraté, appauvri en composés oxygénés, alimentant l'étape D) et en fond un raffinat eau usée. Ledit raffinat eau usée contient des composés polaires solubles comme l'acétaldéhyde et un peu de butadiène, et alimente l'étape E) de traitement des effluents.

### Étape C) de séparation

Le procédé selon l'invention comprend une étape C) de séparation alimentée au moins par ledit effluent éthanol-butadiène issu de l'étape B) et produisant au moins un distillat butadiène et un résidu réactifs pollués.

L'effluent éthanol-butadiène issu de l'étape B) alimente ladite étape C) de séparation de manière à séparer en tête un distillat butadiène comprenant la majorité du butadiène, et en fond un résidu réactifs pollués. Par la majorité, on entend plus de 80% poids du butadiène compris dans l'alimentation de ladite étape C) de séparation, préférentiellement plus de 90%, de manière préférée plus de 95%, de manière encore plus préférée plus de 98%, de manière très préférée plus de 99% et de manière très avantageuse plus de 99,5% poids du butadiène compris dans ladite alimentation de ladite étape C).

Ce résidu réactifs pollués comprend de l'éthanol et de l'acétaldéhyde, et comprend également de l'eau et des sous-produits formés dans l'étape A), comme par exemple le diéthyl éther, l'acétate d'éthyle et les huiles brunes. Ledit résidu réactifs pollués alimente ensuite l'étape E) de traitement des effluents. Ladite étape C) de séparation est opérée à une pression comprise entre 0,1 et 1 MPa et de manière préférée entre 0,2 et 0,5 MPa. Cette étape est avantageusement mis en oeuvre par distillation.

L'agencement des étapes du procédé selon l'invention, en particulier la mise en oeuvre de séparation par distillations extractives dans l'étape D) permet d'être moins sévère sur la quantité de composés oxygénés dans le distillat butadiène. En effet, l'éthanol, l'acétaldéhyde et le diéthyléther peuvent représenter ensemble jusqu'à 60%poids de celui-ci.

### Étape D) de purification du butadiène

Le procédé selon l'invention comprend une étape D) de purification du butadiène comprenant :
- une section de séparation du butène-1 alimentée au moins par l'effluent butadiène hydraté issu de l'étape B), en mélange avec ledit distillat butadiène issu de l'étape C) et produisant un effluent gaz légers et un distillat butadiène étêté ;
- une section de séparation des oxygénés alimentée au moins par ledit distillat butadiène étêté issu de la section de séparation du butène-1 et produisant un effluent butadiène pré-purifié et un effluent composés oxygénés ;
- une section de distillation finale alimentée par ledit effluent butadiène pré-purifié issu de la section de séparation des oxygénés séparant en tête un effluent butadiène purifié et en fond un résidu butène-2.

Ladite étape D) est alimentée par l'effluent butadiène hydraté issu de l'étape B), en mélange avec le distillat butadiène issu de l'étape C). Ledit mélange comprend au moins 50% poids, de préférence au moins 60% pds, de manière préférée au moins 70 % pds, de manière très préférée au moins 80% pds de butadiène ainsi que des impuretés, dues en particulier à la dégradation de la sélectivité en butadiène dans la section réactionnelle, parmi lesquelles on compte des composés oxygénées non désirés dans l'effluent butadiène purifié tels que l'éthanol, l'acétaldéhyde, le diéthyl éther et l'eau, au plus 15% poids d'impuretés en C₄ de type butènes et butanes, des hydrocarbures comprenant au moins 5 atomes de carbone (hydrocarbures C₅⁺) ainsi que des gaz légers, en particulier de l'hydrogène, de l'éthane, de l'éthylène, du propane, du propylène.

Ladite section de séparation du butène-1 comprend une distillation extractive, alimentée par ledit effluent butadiène hydraté, en mélange avec ledit distillat butadiène et par un flux comprenant un solvant, et séparant en tête un effluent gaz légers, et en fond un résidu butadiène.

Par solvant, on entend tout solvant polaire, miscible en phase liquide avec ladite charge butadiène dans les conditions opératoires de ladite distillation extractive, ayant une volatilité plus faible que les composés 1,3-butadiène, butène-2 et butynes, de manière à rester en phase liquide dans ladite section de séparation du butène-1, mais pouvant être séparé de ces composés par distillation. Ledit solvant est avantageusement choisi dans le groupe constitué par le diméthylformamide (DMF), la N-méthylpyrrolidone et l'acétonitrile.

L'effluent butadiène hydraté, en mélange avec ledit distillat butadiène, est avantageusement comprimé dans une section de compression à une pression comprise entre 0,1 et 1,0 MPa, préférentiellement entre 0,1 et 0,7 MPa, et de manière préférée entre 0,2 et 0,5 MPa. L'effet de cette compression est de diminuer le débit volumique de gaz.

Dans le cas préféré où ledit solvant est le DMF, la mélange eau-DMF étant corrosif, la teneur en eau dans l'effluent butadiène hydraté, en mélange avec ledit distillat butadiène, avantageusement comprimé, devra être ajustée en accord avec les contraintes métallurgiques. De plus, les éléments corrodés auront tendance à catalyser la réaction de polymérisation du butadiène, entrainant une perte de rendement et un risque de colmatage des lignes. La teneur en eau peut être réduite par tout moyen connu de l'Homme du métier, par exemple par séchage, avantageusement par séchage sur adsorbant, l'adsorbant pouvant être silicique et/ou aluminique. De manière non limitative, cet adsorbant peut être une zéolite telle qu'une zéolite 3A ou 4A. De manière avantageuse, la teneur en eau dans l'effluent butadiène hydraté, en mélange avec ledit distillat butadiène, avantageusement comprimé, est réduite à une valeur inférieure à 3% poids dudit effluent. Il est également possible d'ajouter un inhibiteur de corrosion connu de l'Homme du métier audit effluent.

L'effluent gaz légers produit en tête de la distillation extractive de ladite section de séparation du butène-1 comprend des butènes 1 et 2, ainsi que des gaz légers tels que l'hydrogène, l'éthane, l'éthylène, le propane, le propylène. Lorsque la teneur en gaz légers dans l'effluent butadiène hydraté, en mélange avec ledit distillat butadiène est importante, par exemple supérieure à 2% du poids total dudit effluent, un refroidissement important de la tête de ladite distillation extractive peut être nécessaire afin d'assurer un reflux suffisant dans ladite colonne. Ce refroidissement est classiquement réalisé en utilisant un groupe froid.

L'effluent gaz légers peut être brulé pour fournir une partie de la chaleur nécessaire au circuit d'huile chaude ou aux chaudières à vapeur du procédé. L'agencement des étapes de procédé selon l'invention permet de produire un effluent gaz légers à un niveau de pression suffisant pour être envoyé vers un four ou vers un système d'élimination par combustion (torche).

De manière avantageuse, ledit effluent butadiène hydraté, en mélange avec ledit distillat butadiène est prélavé par mise en contact avec un flux comprenant un solvant polaire choisi dans le groupe constitué par le diméthylformamide (DMF), la N-méthylpyrrolidone (NMP) et l'acétonitrile préalablement à son alimentation dans ladite section de séparation du butène-1. Ce prélavage permet de séparer la majorité des gaz légers et ainsi de se passer de refroidissement nécessitant la mise en oeuvre d'un groupe froid en tête de ladite distillation extractive.

Ladite distillation extractive est opérée de telle sorte que ledit résidu butadiène comprenne au moins 95% poids, avantageusement au moins 98% poids et de manière préférée au moins 99% poids du butadiène compris dans ledit effluent butadiène hydraté, en mélange avec ledit distillat butadiène. Ladite distillation extractive est également opérée de telle sorte que la quantité de butène-1 dans ledit résidu butadiène représente au plus 0,5% du poids de butadiène compris dans ledit résidu. L'opération est effectuée en ajustant le ratio débit de solvant sur débit d'effluent butadiène, ainsi que le taux de reflux de la distillation extractive, comme connu de l'Homme du métier.

Ladite distillation extractive est opérée à la pression la plus basse possible pour limiter l'exposition des flux riches en butadiène à des hautes températures où une polymérisation ou décomposition pourraient avoir lieu. De préférence, la pression d'opération de ladite section est inférieure à 0,6 MPa, et de manière préférée inférieure à 0,5 MPa.

Ladite section de séparation du butène-1 comprend une également une distillation alimentée par ledit résidu butadiène et séparant en tête un distillat butadiène étêté et en fond un résidu solvant.

La distillation est une distillation classique connue de l'Homme du métier, opérée de telle sorte que ledit résidu solvant comprenne moins de 1% poids de butadiène, avantageusement ne comprenne plus de butadiène, et que ledit distillat butadiène étêté comprenne moins de 0,5% poids de solvant, avantageusement ne comprenne plus de solvant.

Ladite distillation est opérée à la pression la plus basse possible pour limiter l'exposition des flux riches en butadiène à des hautes températures où une polymérisation ou décomposition pourraient avoir lieu. Ladite distillation est avantageusement opérée à une température en tête inférieure à 60°C, de préférence inférieure à 50°C, et à une pression en tête inférieure à 0,5 MPa, de préférence inférieure à 0,4 MPa.

Ledit résidu solvant alimente avantageusement la section de séparation du butène-1 en tant que flux comprenant un solvant, avantageusement en mélange avec un appoint de solvant.

Ladite section de séparation des oxygénés comprend une distillation extractive alimentée par ledit distillat butadiène étêté et par un flux comprenant un solvant et produit en tête un effluent butadiène pré-purifié et en fond un résidu solvant usé.

Ledit solvant est avantageusement choisi dans le groupe constitué par le diméthylformamide (DMF), la N-méthylpyrrolidone (NMP) et l'acétonitrile.

Ladite distillation extractive est opérée de telle sorte que ledit effluent butadiène pré-purifié comprenne au moins 98% poids, avantageusement au moins 99% poids du butadiène compris dans ledit distillat butadiène étêté. Ladite distillation extractive est également opérée de telle sorte que la teneur en alcynes à 4 atomes de carbones présente dans l'effluent butadiène purifié en tête de la section de distillation finale soit conforme aux spécifications exigées pour l'utilisation ultérieure dudit effluent. L'opération est effectuée en ajustant le ratio débit de solvant sur débit de distillat butadiène étêté, ainsi que le taux de reflux de ladite distillation extractive, comme connu de l'Homme du métier.

Ladite distillation extractive est opérée à la pression la plus basse possible pour limiter l'exposition des flux riches en butadiène à des hautes températures où une polymérisation ou décomposition pourraient avoir lieu. De préférence, la pression d'opération de ladite distillation est inférieure à 0,6 MPa, et de manière préférée inférieure à 0,5 MPa.

Ladite section de séparation des oxygénés comprend également une distillation alimentée par ledit résidu solvant usé et séparant en tête un effluent composés oxygénés et en fond un résidu solvant.

La distillation est une distillation classique connue de l'Homme du métier, opérée de telle sorte que ledit résidu solvant comprenne moins de 1% poids de butadiène, avantageusement ne comprenne plus de butadiène, et que la perte en solvant dans ledit effluent composés oxygénés soit inférieure à 0,05% poids, de préférence inférieure à 0,01% poids. Par perte en solvant, on entend le ratio du débit de solvant dans ledit effluent composés oxygénés sur le débit de solvant dans ledit résidu solvant usé. Ledit effluent composés oxygénés comprend donc, outre des composés oxygénés, des alcynes à 4 atomes de carbone.

Ladite distillation est également opérée de telle sorte que la teneur en composés acétyléniques dans ledit effluent composés oxygénés ne dépasse pas 30% poids pour éviter tout risque d'explosion favorisé par une augmentation de la pression et de la température, phénomène connu de l'Homme du métier.

Ladite distillation est avantageusement opérée à une température en tête inférieure à 60°C, de préférence inférieure à 50°C, et à une pression en tête inférieure à 0,5 MPa, de préférence inférieure à 0,4 MPa.

Ledit résidu solvant alimente avantageusement la section de séparation des oxygénés en tant que flux comprenant un solvant, avantageusement en mélange avec un appoint de solvant.

Le résidu solvant issu de la section de séparation du butène-1 et le résidu solvant issu de la section de séparation des oxygénés peuvent avantageusement être mélangés avant d'alimenter la section de séparation du butène-1 et la section de séparation des oxygénés en tant que flux comprenant un solvant. Tout ou partie du résidu solvant issu de la section de séparation du butène-1 et éventuellement de la section de séparation des oxygénés peut avantageusement être envoyé dans une section de purification du solvant, par exemple par distillation ou tout autre opération connu de l'Homme du Métier, pour séparer les impuretés lourdes.

Ladite étape D) comprend une section de distillation finale alimentée par ledit effluent butadiène pré-purifié, séparant en tête un effluent butadiène purifié et en fond un résidu butène-2.

Cette section permet d'éliminer les impuretés lourdes, et notamment les traces de cis-2-butènes, ainsi que les butynes-1 résiduels et le 1,2-butadiène éventuellement présents dans le distillat butadiène étêté.

Ledit effluent butadiène purifié comprend au moins 99,5 % poids de butadiène. Le rendement de l'étape D) de purification selon l'invention, défini comme le débit de butadiène dans l'effluent butadiène purifié sur le débit de butadiène dans l'effluent butadiène hydraté, en mélange avec ledit distillat butadiène alimentant l'étape D) est au moins égal à 95 % poids, de préférence à 98%, de manière préférée supérieur à 99% poids

### Étape E) de traitement des effluents

Le procédé selon l'invention comprend une étape E) de traitement des effluents alimentée au moins par le résidu réactifs pollués issu de l'étape C) et par l'effluent composés oxygénés issu de l'étape D), et produisant au moins un effluent éthanol, un effluent éthanol-acétaldéhyde, un effluent huiles brunes légères et un effluent huiles brunes lourdes.

De préférence, ladite étape E) comprend au moins une section de lavage/contre-lavage, une section de distillation des huiles brunes légères, une section de distillation des huiles brunes lourdes, une section de séparation de l'acétaldéhyde et une section de séparation éthanol-eau.

Ladite section de lavage/contre-lavage préférentielle est alimentée en un point intermédiaire par ledit résidu réactifs pollués issu de l'étape C), en mélange avec ledit effluent composés oxygénés issu de l'étape D), avantageusement en mélange avec l'effluent impuretés issu de l'étape B), avantageusement en mélange avec le résidu oxygénés issu de l'étape B).

Ladite section de lavage/contre-lavage préférentielle est alimentée en fond par un effluent hydrocarbures et en tête par au moins une fraction du résidu eau recyclée issue de la section de séparation éthanol-eau. Ladite section de lavage/contre-lavage peut également être alimentée par un flux d'eau externe au procédé en complément et/ou en remplacement de ladite fraction du résidu eau recyclée. L'effluent hydrocarbures et la fraction du résidu eau recyclée sont alimentés à une température de préférence comprise entre 10 et 70°C, préférentiellement entre 45 et 55°C. Ladite section de lavage/contre-lavage produit en tête un extrait hydrocarbures de lavages chargé d'une fraction des impuretés et des huiles brunes, et en fond un raffinat éthanol / acétaldéhyde / eau.

Ladite section de lavage/contre-lavage est de préférence opérée à une pression comprise entre 0,1 et 0,5 MPa, préférentiellement entre 0,2 et 0,4 MPa. De préférence, l'ajout d'eau pour réaliser le contre-lavage est tel que la teneur en eau dans le raffinat eau / éthanol / acétaldéhyde est supérieure à 30% poids, de manière préférée supérieur à 40% poids.

Dans un mode de réalisation, le contact entre les deux phases liquides dans ladite section de lavage/contre-lavage est réalisé au sein d'un extracteur liquide-liquide. Différents modes de contact peuvent être envisagés. On peut citer de manière non limitative, une colonne garnie, une colonne puisée, ou bien une colonne compartimentée agitée. Dans un autre mode de réalisation, le contact entre les deux phases liquides dans ladite section de lavage/contre-lavage est réalisé au sein d'un contacteur membranaire, ou une cascade de contacteurs membranaires. Ce mode de contact est particulièrement bien adapté au système mis en oeuvre. En effet, les mélanges eau-éthanol-hydrocarbures sont connus pour former des émulsions stables, qui peuvent être problématiques dans un extracteur liquide-liquide. Le contacteur membranaire permet de générer une aire de contact importante, favorisant le transfert des impuretés et des huiles vers la phase hydrocarbure, sans générer d'émulsion.

Ledit extrait hydrocarbures de lavages alimente ladite section de distillation des huiles brunes légères, laquelle produit en tant que distillat ledit effluent huiles brunes légères, et un résidu hydrocarbures comprenant la fraction lourde des huiles brunes.

Ledit effluent huiles brunes légères est composé d'impuretés produites dans la section réactionnelle de l'étape A), principalement du diéthyl éther, d'acétate d'éthyle et du crotonaldéhyde, ainsi que de la fraction légère des huiles brunes, composée d'impuretés en quantité plus faible, parmi lesquelles du pentène, de l'isoprène, du butanal, du vinyl ethyl ether. Cet effluent peut être brulé pour fournir une partie de la chaleur nécessaire au circuit d'huile chaude ou aux chaudières à vapeur du procédé, ou distillé pour récupérer un effluent diéthyl éther et/ou un effluent acétate d'éthyle/crotonaldéhyde, qui pourra être soit valorisée, soit recyclée dans la section réactionnelle de l'étape A) pour être retransformé.

Ledit résidu hydrocarbures contient essentiellement les hydrocarbures servant au lavage, mais également la fraction la plus lourde des huiles brunes. Pour éviter l'accumulation des huiles brunes par le recyclage de l'effluent hydrocarbures vers l'extracteur liquide-liquide, une fraction dudit résidu hydrocarbures est traitée dans ladite section de distillation des huiles lourdes, consistant en une colonne à distiller, laquelle produit un distillat hydrocarbures composé pour l'essentiel d'hydrocarbures avec encore quelques traces d'huiles brunes et, en tant que résidu, ledit effluent huiles brunes lourdes comprenant plus de 80%, préférentiellement plus de 85% d'hydrocarbures ainsi que les huiles brunes les plus lourdes. La fraction dudit effluent hydrocarbures envoyée vers ladite section de distillation des huiles est comprise entre 5 et 30% du débit total dudit résidu hydrocarbures, et préférentiellement entre 10 et 20%. Le distillat hydrocarbures est mélangé à la fraction du résidu hydrocarbures qui n'a pas été traité dans ladite section de distillation des huiles lourdes afin de former l'effluent hydrocarbures alimentant ladite section de lavage/contre-lavage.

Cet effluent, qui représente de préférence entre 0,1 et 20% de la charge de ladite section de distillation des huiles lourdes, préférentiellement entre 0,3 et 5%, peut être brûlé pour fournir une partie de la chaleur nécessaire au circuit d'huile chaude ou aux chaudières à vapeur du procédé. Un appoint d'hydrocarbures équivalent aux pertes en fond de ladite section de distillation des huiles lourdes est nécessaire pour maintenir le débit de lavage constant. Cette colonne est réglée de manière à maintenir constante la concentration en huiles brunes dans la boucle de recyclage des hydrocarbures (boucle effluent hydrocarbures /effluent hydrocarbures de lavages).

Les effluents huiles brunes légères et lourdes sont éliminés du procédé.

Le résidu réactifs pollués issu de l'étape C) comprend principalement de l'éthanol, de l'acétaldéhyde, de l'eau mais aussi des impuretés telles que le diéthyl éther, l'acétate d'éthyle et les huiles brunes telles que définies précédemment. Ces impuretés peuvent s'accumuler si elles sont renvoyées vers l'étape A) au sein de l'effluent éthanol-acétaldéhyde et/ou de l'effluent éthanol et qu'elles ne sont que partiellement converties dans la section réactionnelle de l'étape A). La section de lavage-contre lavage permet de récupérer une partie de ces impuretés avant les sections de séparation de l'acétaldéhyde et de séparation eau-éthanol, ce qui permet d'éviter la démixtion des huiles brunes au sein de ces sections.

Le lavage du résidu réactifs pollués issu de l'étape C) avec un effluent hydrocarbures entraîne certaines impuretés, tandis que le contre-lavage du flux d'hydrocarbures avec une fraction du résidu eau recyclée (ou le cas échéant avec un flux d'eau externe au procédé en complément et/ou en remplacement de ladite fraction du résidu eau recyclée) limite toute perte en acétaldéhyde et en éthanol.

Ledit effluent hydrocarbures peut contenir des hydrocarbures saturés et/ou insaturés et /ou aromatiques, de préférence des hydrocarbures saturés. Ledit effluent hydrocarbures est avantageusement constitué d'un mélange d'hydrocarbures ayant entre 6 et 40 atomes de carbone, de préférence entre 10 et 20 atomes de carbone. De manière non limitative, ledit effluent hydrocarbures peut être une coupe gazole ou kérosène désulfurée ou bien encore une coupe d'hydrocarbures produite par une unité de type Fischer-Tropsh.

L'ajout d'eau au sein de la section de lavage/contre-lavage permet un meilleur fonctionnement du procédé d'élimination des impuretés et des huiles brunes selon l'invention.

Le procédé selon l'invention évite ainsi la purge régulière d'éthanol afin d'éviter l'accumulation des huiles brunes, ce qui permet d'améliorer les performances globales du procédé.

Ledit effluent eau/éthanol/acétaldéhyde issu de la section de lavage-contre lavage alimente ladite section de séparation de l'acétaldéhyde, dans laquelle l'acétaldéhyde est séparé de manière à former un effluent éthanol-acétaldéhyde et un effluent éthanol-eau. Ladite section de séparation met avantageusement en oeuvre une distillation.

L'effluent éthanol-acétaldéhyde issu de l'étape E) est constitué majoritairement d'acétaldéhyde et d'éthanol. Par majoritairement, on entend que l'ensemble éthanol + acétaldéhyde représente plus de 80% pds, de préférence plus de 85 % poids dudit effluent. De manière non limitative, l'effluent éthanol-acétaldéhyde issu de l'étape E) peut contenir des impuretés comme de l'eau, de l'acétate d'éthyle, de l'acétone. Les impuretés autre que l'eau représentent moins de 10%, de façon préférentielles moins de 8% poids du flux.

Dans un mode de réalisation de l'invention, ledit effluent éthanol-acétaldéhyde subit une étape de purification avant d'être recyclés dans le reste du procédé. Par purification, on entend mettre en contact ledit effluent avec des adsorbants comme par exemple du charbon actif, de la silice, de l'alumine ou encore une résine polymérique fonctionnalisée.

Ledit effluent éthanol-eau issu de ladite section de séparation de l'acétaldéhyde alimente la section de séparation éthanol-eau dans laquelle il est séparé en un effluent éthanol et un résidu eau recyclée.

Ladite section de séparation éthanol-eau est avantageusement réalisée par distillation. Au moins une fraction de la charge éthanol du procédé selon l'invention peut avantageusement être introduite en tête de ladite distillation, ce qui a pour effet de faciliter la distillation de l'éthanol en présence d'impuretés, de réduire le reflux et d'augmenter la concentration en éthanol dans l'effluent éthanol alimentant la section réactionnelle et donc de baisser le débit total de cet effluent pour un même débit d'éthanol, ce qui permet de travailler avec une section réactionnelle de plus faible volume que dans l'art antérieur, dans lequel la charge éthanol est d'abord utilisée pour solubiliser le butadiène dans l'effluent vapeur de la section réactionnelle.

Cette utilisation de la charge éthanol selon l'art antérieur a pour avantage de permettre d'extraire les composés les plus légers compris dans l'effluent de la section réactionnelle, ces composés étant séparés par lavages successifs avec la charge éthanol et avec un flux d'eau. Dans le procédé selon l'invention, ces composés légers restent avec le butadiène jusqu'à l'étape de purification du butadiène, ce qui a pour conséquence d'augmenter la taille des équipements et peut, en fonction de la concentration en composés légers, imposer d'utiliser une étape de pré-lavage ou des groupes froids en tête des sections de séparation du butène-1 afin d'assurer la liquéfaction d'une partie du distillat et d'assurer un reflux. Ce choix est donc à priori néfaste aux performances du procédé. Or, la demanderesse s'est aperçue que l'alimentation de la charge éthanol en tête d'une section de séparation eau-éthanol en association avec une étape de purification du butadiène réalisée par distillation extractive et plus apte à gérer les composés légers, malgré l'envoi des composés légers dans l'étape C) de séparation (ayant à priori un impact négatif car plus énergivore) aboutissait au global à une amélioration des performances du procédé selon l'invention, en particulier par l'amélioration du fonctionnement de la section de séparation eau-éthanol et par la minimisation de réaction éthanol-acétaldéhyde dans la section de lavage-contre lavage.

Dans un mode de réalisation de l'invention, l'effluent éthanol subit une étape de purification avant d'alimenter l'étape A) de conversion. Par purification, on entend mettre en contact ledit effluent avec des adsorbants comme par exemple du charbon actif, de la silice, de l'alumine ou encore une résine polymérique fonctionnalisée. Par exemple, un charbon actif permet d'éliminer les traces de butanol et d'hexanol.

L'effluent éthanol comprend majoritairement de l'éthanol. Par majoritairement, on entend que l'éthanol représente plus de 80% poids, de préférence plus de 84% poids dudit effluent. De manière non limitative, l'effluent riche en éthanol peut contenir des impuretés comme de l'eau, de l'acétate d'éthyle, du butanol et de l'hexanol. Les impuretés autre que l'eau représentent moins de 10%, de façon privilégiée moins de 5%, de façon encore préférentielle moins de 2% poids dudit effluent.

### DESCRIPTION DES FIGURES

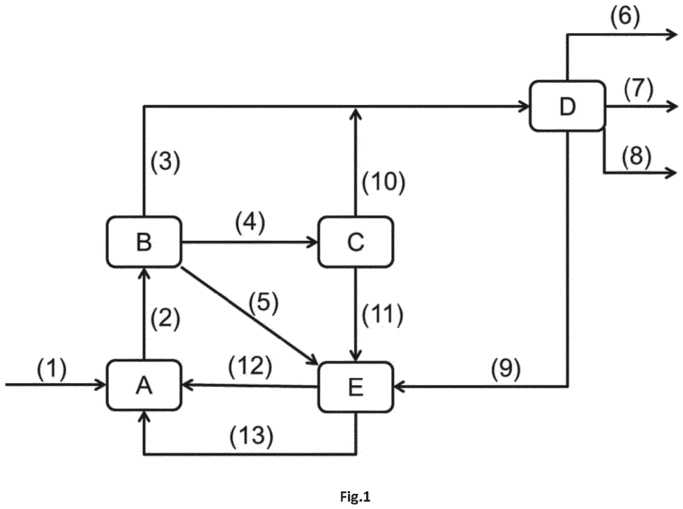

La figure 1 présente de manière schématique une vue générale d'un arrangement du procédé selon l'invention.

Une étape de **A** de conversion de l'éthanol en butadiène est alimentée par une charge éthanol (1), ainsi que par l'effluent éthanol (13) et par l'effluent éthanol-acétaldéhyde (12) issus de l'étape **E.** Cette étape produit un effluent réactionnel (2) qui alimente une étape **B** de séparation des réactifs, laquelle produit un effluent éthanol-butadiène (4), un effluent impuretés (5) et un effluent butadiène hydraté (3). Dans l'arrangement présenté figure 1, l'effluent impuretés (5) alimente l'étape **E** de traitement des effluents.

L'effluent éthanol-butadiène (4) alimente l'étape **C** de séparation de manière à produire un distillat butadiène (10) et un résidu réactifs pollués (11).

Le distillat butadiène (10), en mélange avec l'effluent butadiène hydraté (3) alimentent l'étape **D** de purification du butadiène. L'étape **D** produit un effluent gaz légers (6), un effluent composés oxygénés (9), un résidu butène-2 (8) et, en tant que produit du procédé, un effluent butadiène purifié (7).

L'effluent composés oxygénés (9) et le résidu réactifs pollués (11) alimentent l'étape **E** de traitement des effluents de manière à produire un effluent éthanol (13), un effluent éthanol-acétaldéhyde (12), un effluent huiles brunes légères et un effluent huiles brunes lourdes (non représentés).

### EXEMPLES

Les exemples suivants sont basés sur des simulations intégrant les recyclages des flux. Dans chacun des exemples, le débit de charge éthanol est ajusté de manière à obtenir une production annuelle de 150 kt/an d'un effluent butadiène purifié ayant une pureté comprise entre 99,5 et 100 % poids.

Tous les pourcentages sont des pourcentages poids.

### Exemple 1 - Comparatif

Un procédé de production de butadiène à partir d'éthanol conforme au procédé décrit dans WO2016/042095 est alimenté par 48,2 t/h d'une charge éthanol comprenant 93,4% poids d'éthanol. Ce schéma ne met pas en oeuvre de distillation extractive et la charge éthanol est utilisée pour laver l'effluent réactionnel.

La charge éthanol est utilisée pour laver l'effluent vapeur issu de la section réactionnelle, après que celui-ci a été séparé de sa partie liquide et comprimé.

La charge éthanol issue de la section de lavage est mélangée à la partie liquide de l'effluent de la section réactionnelle, formant un flux de 166 t/h comprenant 54% d'éthanol, 4% d'acétaldéhyde, 23% d'eau et 11% de butadiène.

Ce flux est traité dans une section de distillation produisant en tête 20 t/h d'un distillat comprenant le butadiène et 146 t/h d'un résidu ne comprenant plus de butadiène, et comprenant 61% d'éthanol, 4% d'acétaldéhyde et 27% d'eau.

Le distillat comprenant le butadiène est traité successivement par lavage à l'eau, séchage, distillation cryogénique et séparation au DMSO afin de produire 18,75 t/h d'un effluent butadiène purifié comprenant 99,6% de butadiène. Le lavage à l'eau met en oeuvre 21 t/h d'eau d'origine externe au procédé. L'eau issue du lavage du distillat comprenant le butadiène alimente la section de séparation de l'acétaldéhyde.

L'effluent vapeur lavé par la charge éthanol est lavé par de l'eau issue du traitement des effluents afin de capter les traces d'éthanol et d'acétaldéhyde. L'eau issue de ce lavage (environ 0,4 t/h) alimente une section de séparation eau-éthanol.

Les 146 t/h de résidu sont traités dans une section de lavage-contre lavage aux hydrocarbures afin de séparer les impuretés, et en particulier les huiles brunes. L'effluent de cette section, représentant 168 t/h et comprenant 53% d'éthanol, 4% d'acétaldéhyde et 40% d'eau, alimente deux colonnes à distiller permettant de séparer un effluent éthanol-acétaldéhyde, un effluent éthanol et un effluent eau, lequel est en partie (29,5 t/h) recyclé vers le lavage de l'effluent vapeur issu de la section réactionnelle (0,4 t/h) et vers la section de lavage-contre lavage (29,1 t/h), et en partie (41,4 t/h) purgé.

L'effluent éthanol (95,7 t/h comprenant 79% d'éthanol) et l'effluent éthanol-acétaldéhyde (23,2 t/h comprenant 57% d'éthanol et 27% d'acétaldéhyde) alimentent la section réactionnelle.

La consommation en énergie de ce schéma en terme d'utilités (électricité, gaz et vapeur) exprimée en MWh est de 178,8 MWh.

### Exemple 2 - Conforme à l'invention

Un procédé de production de butadiène à partir d'éthanol conforme au procédé selon l'invention est alimenté par 48,2 t/h d'une charge éthanol comprenant 93,4% poids d'éthanol.

Conformément à l'invention, la charge éthanol n'est pas utilisée pour laver l'effluent vapeur issu de la section réactionnelle. La charge éthanol est alimentée en tête de la section de séparation éthanol-eau, réalisée dans une colonne à distiller. Cette section de séparation produit en fond 39 t/h d'eau (dont 18 t/h sont recyclées vers la section de séparation des huiles brunes et dont 21 t/h sont purgées du procédé) et en tête 90 t/h d'effluent éthanol alimentant la section réactionnelle.

La section réactionnelle comprend deux zones réactionnelles. La première est alimentée par 75 t/h de l'effluent éthanol. L'effluent de la première zone réactionnelle est séparé en un effluent gaz et un effluent liquide. L'effluent gaz (environ 11 t/h) est lavé par les 15 t/h de l'effluent éthanol n'alimentant pas la première zone réactionnelle. Le gaz lavé (1 t/h) comprend principalement de l'hydrogène. L'effluent éthanol ayant lavé l'effluent gaz, en mélange avec l'effluent liquide et l'effluent éthanol-acétaldéhyde issu de l'étape de traitement des effluents, alimente la seconde zone réactionnelle avec un débit total de 112 t/h.

L'effluent réactionnel issu de la seconde zone réactionnelle forme un flux de 112 t/h comprenant 39% d'éthanol, 6% d'acétaldéhyde, 28% d'eau et 17% de butadiène.

Ce flux est traité dans un flash à 75°C, produisant 33 t/h d'un effluent liquide, contenant majoritairement de l'eau, de l'éthanol et des impuretés lourdes (mais ne contenant plus de butadiène) qui alimente directement la section de traitement des huiles. Le flash produit également un effluent gazeux qui, après compression, alimente un second flash à 45°C. L'effluent liquide et gazeux de ce second flash sont envoyés pour le premier dans une étape de séparation (étape C) du procédé selon l'invention) et pour le second dans une section de distillation.

Les distillats de l'étape de séparation et de la section de distillation, dont les débits sont respectivement de 9,5 et 11,5 t/h sont envoyés en mélange vers une étape de séchage, puis une section de refroidissement et compression.

Les résidus de l'étape de séparation et de la section de distillation, dont les débits sont respectivement de 57 et 1 t/h, ne contenant plus de butadiène, sont envoyés en mélange vers la section de lavage - contre lavage des effluents aux hydrocarbures.

Le mélange envoyé vers une étape de séchage, puis une section de refroidissement et compression, contenant 90 % de butadiène, 7 % de légers et 3 % d'acétaldéhyde, est ensuite envoyé vers une section de séparation du butène-1 par distillation extractive qui permet de soutirer en tête un effluent gaz légers, comprenant principalement les gaz éthylène, propylène, butène 1, Trans-2-butène et Cis-2 butènes. Le fond de la colonne de distillation extractive alimente une distillation permettant de séparer le solvant de distillation extractive, la tête de cette colonne, qui forme le distillat butadiène étêté, alimentant une section de séparation des oxygénés. Ladite section de séparation des oxygénés permet d'extraire 0,65 t/h d'un effluent composés oxygénés contenant les impuretés (au sens indésirables dans l'effluent butadiène purifié) oxygénées, dont la totalité de l'acétaldéhyde, effluent qui est envoyé ensuite dans la section de lavage - contre lavage des effluents aux hydrocarbures.

La section de distillation finale conduit à la production d'un effluent de 18,75 t/h de butadiène purifié comprenant 99,6% poids de butadiène.

Le mélange envoyé vers la section de lavage - contre lavage des effluents aux hydrocarbures, contenant au global 48 % d'éthanol, 34 % d'eau , 7 % d'acétaldéhyde (le reste étant constitué d'impuretés huiles brunes), alimente en un point intermédiaire une section de lavage-contre lavage aux hydrocarbures dans une étape de traitement des effluents afin de séparer les impuretés, et en particulier les huiles brunes. Cette section est alimentée en tête par un débit d'eau recyclé de 18 t/h afin de réaliser l'opération de contre-lavage. L'effluent de cette section, représentant 104 t/h et comprenant 42% d'éthanol, 6% d'acétaldéhyde et 47% d'eau, alimente une section de séparation de l'acétaldéhyde permettant de séparer un effluent éthanol-acétaldéhyde et un effluent éthanol-eau. L'effluent éthanol-acétaldéhyde (23 t/h comprenant 56% d'éthanol et 27% d'acétaldéhyde) alimente la seconde zone réactionnelle.

L'effluent éthanol-eau alimente la section de séparation éthanol-eau en un point intermédiaire, section par ailleurs alimentée en tête par la charge éthanol comme indiqué en début d'exemple.

Par rapport au procédé non-conforme, un rendement global en butadiène similaire est obtenu (même débit d'effluent butadiène purifié pour un même débit de charge).

La consommation en énergie de ce schéma en terme d'utilités (électricité, gaz et vapeur) exprimée en MWh est de 138,7 MWh, soit une réduction de 23% de la consommation d'utilités. Ce gain est en partie dû à une meilleure gestion du circuit d'eau et d'éthanol. La réduction du débit d'eau circulant (de 21 T) dans le procédé induit une baisse de consommation au niveau des opérations de séparation. Le choix du point d'injection de la charge éthanol allège également le travail des opérations de séparation. La charge éthanol n'étant pas utilisée pour laver les effluents réactionnels, elle n'est plus traitée dans les étapes de traitement des effluents. Ces réductions de débit ont donc également pour conséquence de diminuer la taille des équipements.

## Revendications

1. Procédé de production de butadiène à partir d'une charge éthanol comprenant au moins 80% poids d'éthanol, ledit procédé comprenant :
A) une étape de conversion de l'éthanol en butadiène comprenant au moins une section réactionnelle alimentée par au moins une fraction de ladite charge éthanol par au moins une fraction de l'effluent éthanol issu de l'étape E), opérée à une pression comprise entre 0,1 et 1,0 MPa et à une température comprise entre 200 et 500°C en présence d'un catalyseur, et produisant au moins un effluent réactionnel ;
B) une étape de séparation des réactifs alimentée au moins par ledit effluent réactionnel et produisant au moins un effluent éthanol-butadiène, un effluent impuretés et un effluent butadiène hydraté ;
C) une étape de séparation alimentée au moins par ledit effluent éthanol-butadiène et produisant au moins un distillat butadiène et un résidu réactifs pollués ;
D) une étape de purification du butadiène comprenant :
- une section de séparation du butène-1 comprenant une distillation extractive, alimentée par ledit effluent butadiène hydraté issu de l'étape B), en mélange avec ledit distillat butadiène issu de l'étape C) et par un flux comprenant un solvant, et séparant en tête un effluent gaz légers, et en fond un résidu butadiène et comprenant également une distillation alimentée par ledit résidu butadiène et séparant en tête un distillat butadiène étêté et en fond un résidu solvant ;
- une section de séparation des oxygénés comprenant une distillation extractive alimentée par ledit distillat butadiène étêté issu de la section de séparation du butène-1 et par un flux comprenant un solvant et produisant en tête un effluent butadiène pré-purifié et en fond un résidu solvant usé et comprenant également une distillation alimentée par ledit résidu solvant usé et séparant en tête un effluent composés oxygénés et en fond un résidu solvant ;
- une section de distillation finale alimentée par ledit effluent butadiène pré-purifié issu de la section de séparation des oxygénés séparant en tête un effluent butadiène purifié et en fond un résidu butène-2 ;
E) une étape traitement des effluents alimentée au moins par le résidu réactifs pollués issu de l'étape C) et par l'effluent composés oxygénés issu de l'étape D), et produisant au moins un effluent éthanol, un effluent éthanol-acétaldéhyde, un ou plusieurs effluents huiles brunes.

2. Procédé selon la revendication 1 dans lequel ladite section réactionnelle de ladite étape a) comprend deux zones réactionnelles, la première alimentée par une fraction dudit effluent éthanol et éventuellement une partie de ladite charge éthanol, et la seconde alimentée par l'effluent de la première zone réactionnelle, par la fraction résiduelle dudit effluent éthanol et par une fraction de l'effluent éthanol-acétaldéhyde issu de l'étape e) et éventuellement par une partie de ladite charge éthanol, le rapport molaire éthanol sur acétaldéhyde en entrée de ladite seconde zone réactionnelle étant compris entre 1 et 5.

3. Procédé selon la revendication 2 dans lequel un moyen de séparation gaz-liquide est mis en oeuvre entre les deux zones réactionnelles pour séparer l'effluent de la première section réactionnelle en un effluent gaz et un effluent liquide, l'effluent liquide alimentant la seconde zone réactionnelle.

4. Procédé selon l'une des revendications précédentes dans lequel ladite étape B) comprend :
- une section de séparation gaz-liquide séparant ledit effluent réactionnel en un effluent liquide constituant ledit effluent éthanol-butadiène et un effluent gaz,
- une section de compression comprimant ledit effluent gaz en un effluent gaz comprimé à une pression comprise entre 0,1 et 1,0 MPa,
- une section de distillation alimentée par ledit effluent gaz comprimé et séparant en tête ledit effluent butadiène hydraté et en fond ledit effluent impuretés.

5. Procédé selon la revendication 4 dans lequel ladite section de séparation gaz-liquide de ladite étape B) est opérée à une température comprise entre 10 et 20°C.

6. Procédé selon la revendication 4 dans lequel ladite section de séparation de ladite étape B) est opérée à une température comprise entre 40 et 50°C.

7. Procédé selon l'une des revendications 4 à 6 dans lequel ledit effluent impuretés issu de l'étape B) est envoyé vers l'étape E) de traitement des effluents en mélange avec le résidu réactifs pollués issu de l'étape C).

8. Procédé selon l'une des revendications 1 à 3 dans lequel ladite étape B) comprend :
- une première section de séparation gaz-liquide séparant ledit effluent réactionnel en un effluent liquide constituant ledit effluent impuretés et un effluent gaz, opérée à une température comprise entre 70 et 80°C, et une pression comprise entre 0,1 et 0,3 MPa,
- une section de compression comprimant ledit effluent gaz en un effluent gaz comprimé à une pression comprise entre 0,1 et 1,0 MPa,
- une seconde section de séparation gaz-liquide alimentée par ledit effluent gaz comprimé et séparant en tant que phase gaz ledit effluent butadiène hydraté et en tant que phase liquide ledit effluent éthanol-butadiène, opérée à une température comprise entre 40 et 50°C et une pression comprise entre 0,2 et 0,6 MPa,
ledit effluent impuretés alimentant ladite étape E).

9. Procédé selon la revendication 8 dans lequel ledit effluent butadiène hydraté issu de ladite seconde section de séparation gaz-liquide alimente une section de distillation séparant dudit effluent butadiène hydraté en fond un résidu oxygénés et en tête un effluent butadiène hydraté appauvri en composés oxygénés, ledit effluent butadiène hydraté appauvri en composés oxygénés alimentant alors l'étape D) en tant qu'effluent butadiène hydraté.

10. Procédé selon la revendication 9 dans lequel ledit résidu oxygénés alimente ladite étape E) de traitement des effluents en mélange avec ledit résidu réactifs pollués issu de l'étape C).

11. Procédé selon l'une des revendications 4 à 7 dans lequel ledit effluent butadiène hydraté obtenu à l'issu de ladite section de distillation alimente une section de lavage à l'eau comprenant au moins une section de lavage gaz-liquide alimentée en fond par ledit effluent butadiène hydraté et en tête par un flux d'eau, et produisant en tête un effluent butadiène hydraté, appauvri en composés oxygénés, alimentant l'étape D) et en fond un raffinat eau usée.

12. Procédé selon la revendication 8 dans lequel ledit effluent butadiène hydraté obtenu à l'issu de ladite seconde section de séparation gaz-liquide, alimente une section de lavage à l'eau comprenant au moins une section de lavage gaz-liquide alimentée en fond par ledit effluent butadiène hydraté et en tête par un flux d'eau, et produisant en tête un effluent butadiène hydraté, appauvri en composés oxygénés, alimentant l'étape D) et en fond un raffinat eau usée.

13. Procédé selon l'une des revendications précédentes dans lequel ledit solvant de ladite section de séparation du butène-1 est choisi dans le groupe constitué par le diméthylformamide (DMF), la N-méthylpyrrolidone et l'acétonitrile.

14. Procédé selon l'une des revendications précédentes dans lequel le résidu solvant issu de la section de séparation du butène-1 et le résidu solvant issu de la section de séparation des oxygénés sont mélangés avant d'alimenter la section de séparation du butène-1 et la section de séparation des oxygénés en tant que flux comprenant un solvant.

15. Procédé selon l'une des revendications précédentes dans lequel ladite étape E) comprend au moins une section de lavage/contre-lavage, une section de distillation des huiles brunes légères, une section de distillation des huiles brunes lourdes, une section de séparation de l'acétaldéhyde et une section de séparation éthanol-eau.

## Patentansprüche

1. Verfahren zur Herstellung von Butadien aus einem Ethanol-Einsatzstoff, der mindestens 80 Gewichts-% Ethanol umfasst, wobei das Verfahren umfasst:
A) einen Schritt zur Umsetzung von Ethanol zu Butadien, der mindestens einen Reaktionsabschnitt umfasst, dem mindestens ein Anteil des Ethanol-Einsatzstoffs und mindestens ein Anteil des Ethanol-Abstroms aus Schritt E) zugeführt werden und der bei einem Druck zwischen 0,1 und 1 MPa und bei einer Temperatur zwischen 200 und 500°C in Gegenwart eines Katalysators betrieben wird und in dem mindestens ein Reaktionsabstrom erzeugt wird;
B) einen Schritt zur Abtrennung der Reaktanten, dem mindestens der Reaktionsabstrom zugeführt wird und in dem mindestens ein Ethanol-Butadien-Abstrom, ein Abstrom aus Verunreinigungen und ein Abstrom aus hydratisiertem Butadien erzeugt werden;
C) einen Abtrennungsschritt, dem mindestens der Ethanol-Butadien-Abstrom zugeführt wird und in dem mindestens ein Butadiendestillat und ein Rückstand aus verunreinigten Reaktanten erzeugt wird;
D) einen Schritt zur Reinigung von Butadien, umfassend:
- einen Abschnitt zur Abtrennung von Buten-1, der eine extraktive Destillation umfasst, dem der Abstrom aus hydratisiertem Butadien aus Schritt B), vermischt mit dem Butadiendestillat aus Schritt C), und ein Strom zugeführt werden, der ein Lösungsmittel umfasst, und in dem am Kopf ein Abgasstrom aus leichten Gasen und am Boden ein Butadienrückstand abgetrennt werden, und der zudem eine Destillation umfasst, der der Butadienrückstand zugeführt wird und in dem am Kopf ein abdestilliertes leichtes Butadiendestillat und am Boden ein Lösungsmittelrückstand abgetrennt werden;
- einen Abschnitt zur Abtrennung von sauerstoffhaltigen Verbindungen, der eine extraktive Destillation umfasst, dem das abdestillierte leichte Butadiendestillat aus dem Abschnitt zur Abtrennung von Buten-1 und ein Strom zugeführt werden, der ein Lösungsmittel umfasst, und in dem am Kopf ein vorgereinigter Butadien-Abstrom und am Boden ein Rückstand aus verbrauchtem Lösungsmittel erzeugt werden, und der zudem eine Destillation umfasst, der der Rückstand aus verbrauchtem Lösungsmittel zugeführt wird und bei der am Kopf ein Abstrom aus sauerstoffhaltigen Verbindungen und am Boden ein Lösungsmittelrückstand abgetrennt werden;
- einen Enddestillationsabschnitt, dem der vorgereinigte Butadien-Abstrom aus dem Abschnitt zur Abtrennung von sauerstoffhaltigen Verbindungen zugeführt wird und in dem am Kopf ein Abstrom aus gereinigtem Butadien und am Boden ein Rückstand aus Buten-2 abgetrennt werden;
E) einen Abstrom-Behandlungsschritt, dem mindestens der Rückstand aus verunreinigten Reaktanten aus Schritt C) und der Abstrom aus sauerstoffhaltigen Verbindungen aus Schritt D) zugeführt werden und in dem mindestens ein Ethanol-Abstrom, ein Ethanol-Acetaldehyd-Abstrom, ein oder mehrere Braunöl-Abströme erzeugt werden.

2. Verfahren nach Anspruch 1, wobei der Reaktionsabschnitt von Schritt a) zwei Reaktionszonen umfasst, wobei der ersten Zone ein Anteil des Ethanol-Abstroms und gegebenenfalls ein Teil des Ethanol-Einsatzstoffs zugeführt werden, und wobei der zweiten Zone der Abstrom der ersten Reaktionszone, der Restanteil des Ethanol-Abstroms und ein Anteil des Ethanol-Acetaldehyd-Abstroms aus Schritt e) und gegebenenfalls ein Teil des Ethanol-Einsatzstoffs zugeführt werden, wobei das Molverhältnis Ethanol zu Acetaldehyd am Eintritt der zweiten Reaktionszone zwischen 1 und 5 beträgt.

3. Verfahren nach Anspruch 2, wobei ein Mittel zur Gas-Flüssigkeits-Trennung zwischen den beiden Reaktionszonen vorgesehen ist, damit der Abstrom des ersten Reaktionsabschnitts in einen Abgasstrom und einen flüssigen Abstrom getrennt wird, wobei der flüssige Abstrom der zweiten Reaktionszone zugeführt wird.

4. Verfahren nach einem der vorhergehenden Ansprüche, wobei Schritt B) umfasst:
- einen Abschnitt zur Gas-Flüssigkeits-Trennung, in dem der Reaktionsabstrom in einen flüssigen Abstrom, der den Ethanol-Butadien-Abstrom bildet, und einen Abgasstrom getrennt wird,
- einen Verdichtungsabschnitt, in dem der Abgasstrom zu einem verdichteten Abgasstrom bei einem Druck zwischen 0,1 und 1,0 MPa verdichtet wird,
- einen Destillationsabschnitt, dem der verdichtete Abgasstrom zugeführt wird und in dem am Kopf der Abstrom aus hydratisiertem Butadien und am Boden der Abstrom aus Verunreinigungen abgetrennt werden.

5. Verfahren nach Anspruch 4, wobei der Abschnitt zur Gas-Flüssigkeits-Trennung von Schritt B) bei einer Temperatur zwischen 10 und 20°C betrieben wird.

6. Verfahren nach Anspruch 4, wobei der Abtrennungsabschnitt von Schritt B) bei einer Temperatur zwischen 40 und 50°C betrieben wird.

7. Verfahren nach einem der Ansprüche 4 bis 6, wobei der Abstrom aus Verunreinigungen aus Schritt B) gemischt mit dem Rückstand aus verunreinigten Reaktanten aus Schritt C) zum Abstrom-Behandlungsschritt E) geleitet wird.

8. Verfahren nach einem der Ansprüche 1 bis 3, wobei Schritt B) umfasst:
- einen ersten Abschnitt zur Gas-Flüssigkeits-Trennung, in dem der Reaktionsabstrom in einen flüssigen Abstrom, der den Abstrom aus Verunreinigungen bildet, und einen Abgasstrom getrennt wird und der bei einer Temperatur zwischen 70 und 80°C und einem Druck zwischen 0,1 und 0,3 MPa betrieben wird,
- einen Verdichtungsabschnitt, in dem der Abgasstrom zu einem verdichteten Abgasstrom bei einem Druck zwischen 0,1 und 1,0 MPa verdichtet wird,
- einen zweiten Abschnitt zur Gas-Flüssigkeits-Trennung, dem der verdichtete Abgasstrom zugeführt wird und in dem als Gasphase der Abstrom aus hydratisiertem Butadien und als flüssige Phase der Ethanol-Butadien-Abstrom abgetrennt werden und der bei einer Temperatur zwischen 40 und 50°C und einem Druck zwischen 0,2 und 0,6 MPa betrieben wird,
wobei der Abstrom aus Verunreinigungen Schritt E) zugeführt wird.

9. Verfahren nach Anspruch 8, wobei der Abstrom aus hydratisiertem Butadien aus dem zweiten Abschnitt zur Gas-Flüssigkeits-Trennung einem Destillationsabschnitt zugeführt wird, in dem von dem Abstrom aus hydratisiertem Butadien am Boden ein Rückstand aus sauerstoffhaltigen Verbindungen und am Kopf ein Abstrom aus hydratisiertem Butadien mit weniger sauerstoffhaltigen Verbindungen abgetrennt werden, wobei der Abstrom aus hydratisiertem Butadien mit weniger sauerstoffhaltigen Verbindungen dann Schritt D) als Abstrom aus hydratisiertem Butadien zugeführt wird.

10. Verfahren nach Anspruch 9, wobei der Rückstand aus sauerstoffhaltigen Verbindungen gemischt mit dem Rückstand aus verunreinigten Reaktanten aus Schritt C) dem Abstrom-Behandlungsschritt E) zugeführt wird.

11. Verfahren nach einem der Ansprüche 4 bis 7, wobei der Abstrom aus hydratisiertem Butadien, der am Ende des Destillationsabschnitts gewonnen wird, einem Abschnitt zum Spülen mit Wasser zugeführt wird, der mindestens einen Gas-Flüssigkeits-Spülabschnitt umfasst, dem am Boden der Abstrom aus hydratisiertem Butadien und am Kopf ein Wasserstrom zugeführt werden und in dem am Kopf ein Abstrom aus hydratisiertem Butadien mit weniger sauerstoffhaltigen Verbindungen, der Schritt D) zugeführt wird, und am Boden Abwasser als Raffinat erzeugt werden.

12. Verfahren nach Anspruch 8, wobei der Abstrom aus hydratisiertem Butadien, der am Ende des zweiten Abschnitts der Gas-Flüssigkeits-Trennung erhalten wird, einem Abschnitt zum Spülen mit Wasser zugeführt wird, der mindestens einen Gas-Flüssigkeits-Spülabschnitt umfasst, dem am Boden der Abstrom aus hydratisiertem Butadien und am Kopf ein Wasserstrom zugeführt werden und in dem am Kopf ein Abstrom aus hydratisiertem Butadien mit weniger sauerstoffhaltigen Verbindungen, der Schritt D) zugeführt wird, und am Boden Abwasser als Raffinat erzeugt werden.

13. Verfahren nach einem der vorhergehenden Ansprüche, wobei das Lösungsmittel von dem Abschnitt zur Abtrennung von Buten-1 aus der Gruppe ausgewählt ist, die aus Dimethylformamid (DMF), N-Methylpyrrolidon und Acetonitril besteht.

14. Verfahren nach einem der vorhergehenden Ansprüche, wobei der Lösungsmittelrückstand aus dem Abschnitt zur Abtrennung von Buten-1 und der Lösungsmittelrückstand aus dem Abschnitt zur Abtrennung der sauerstoffhaltigen Verbindungen gemischt werden, bevor sie dem Abschnitt zur Abtrennung von Buten-1 und dem Abschnitt zur Abtrennung der sauerstoffhaltigen Verbindungen als Strom, der ein Lösungsmittel umfasst, zugeführt werden.

15. Verfahren nach einem der vorhergehenden Ansprüche, wobei Schritt E) mindestens einen Abschnitt Spülung/Rückspülung, einen Abschnitt zur Destillation von leichten Braunölen, einen Abschnitt zur Destillation von schweren Braunölen, einen Schritt zur Abtrennung von Acetaldehyd und einen Abschnitt zur Ethanol-Wasser-Trennung umfasst.

## Claims

1. Process for producing butadiene from an ethanol feedstock comprising at least 80% by weight of ethanol, said process comprising:
A) a step of converting ethanol into butadiene comprising at least one reaction section fed with at least one fraction of said ethanol feedstock and with at least one fraction of the ethanol effluent resulting from step E), performed at a pressure of between 0.1 and 1.0 MPa and at a temperature of between 200°C and 500°C in the presence of a catalyst, and producing at least one reaction effluent;
B) a step of separating the reagents which is fed at least with said reaction effluent and which produces at least an ethanol-butadiene effluent, an impurities effluent and a hydrated butadiene effluent;
C) a separation step fed at least with said ethanol-butadiene effluent and producing at least a butadiene distillate and a contaminated reagents residue;
D) a step of purifying the butadiene comprising:
- a section for separating the 1-butene comprising an extractive distillation, fed with said hydrated butadiene effluent resulting from step B), as a mixture with said butadiene distillate resulting from step C) and with a stream comprising a solvent, and separating, at the top, a light gas effluent, and, at the bottom, a butadiene residue, and also comprising a distillation fed with said butadiene residue and separating, at the top, a topped butadiene distillate and, at the bottom, a solvent residue;
- a section for separating the oxygenated compounds comprising an extractive distillation fed with said topped butadiene distillate resulting from the 1-butene separation section and with a stream comprising a solvent and producing, at the top, a prepurified butadiene effluent, and, at the bottom, a spent solvent residue and also comprising a distillation fed with said spent solvent residue and separating, at the top, an oxygenated compounds effluent and, at the bottom, a solvent residue;
- a final distillation section fed with said prepurified butadiene effluent resulting from the section for separating the oxygenated compounds, separating, at the top, a purified butadiene effluent and, at the bottom, a 2-butene residue;
E) an effluent treatment step fed at least with the contaminated reagents residue resulting from step C) and with the oxygenated compounds effluent resulting from step D), and producing at least an ethanol effluent, an ethanol-acetaldehyde effluent, and one or more brown oil effluents.

2. Process according to Claim 1, in which said reaction section of said step A) comprises two reaction zones, the first fed with a fraction of said ethanol effluent and optionally a portion of said ethanol feedstock, and the second fed with the effluent from the first reaction zone, with the residual fraction of said ethanol effluent and with a fraction of the ethanol-acetaldehyde effluent resulting from step E) and optionally with a portion of said ethanol feedstock, the ethanol/acetaldehyde mole ratio at the inlet of said second reaction zone being between 1 and 5.

3. Process according to Claim 2, in which a gas-liquid separation means is used between the two reaction zones to separate the effluent from the first reaction section into a gas effluent and a liquid effluent, the liquid effluent feeding the second reaction zone.

4. Process according to one of the preceding claims, in which said step B) comprises:
- a gas-liquid separation section which separates said reaction effluent into a liquid effluent constituting said ethanol-butadiene effluent and a gas effluent,
- a compression section which compresses said gas effluent to give a gas effluent compressed to a pressure of between 0.1 and 1.0 MPa,
- a distillation section fed with said compressed gas effluent and which separates, at the top, said hydrated butadiene effluent and, at the bottom, said impurities effluent.

5. Process according to Claim 4, in which said gas-liquid separation section of said step B) is operated at a temperature of between 10°C and 20°C.

6. Process according to Claim 4, in which said separation section of said step B) is operated at a temperature of between 40°C and 50°C.

7. Process according to one of Claims 4 to 6, in which said impurities effluent resulting from step B) is sent to the effluent treatment step E) as a mixture with the contaminated reagents residue resulting from step C).

8. Process according to one of Claims 1 to 3, in which said step B) comprises:
- a first gas-liquid separation section which separates said reaction effluent into a liquid effluent constituting said impurities effluent and a gas effluent, operated at a temperature of between 70°C and 80°C, and a pressure of between 0.1 and 0.3 MPa,
- a compression section which compresses said gas effluent to give a gas effluent compressed to a pressure of between 0.1 and 1.0 MPa,
- a second gas-liquid separation section fed with said compressed gas effluent and separating, as gas phase, said hydrated butadiene effluent and, as liquid phase, said ethanol-butadiene effluent, operated at a temperature of between 40°C and 50°C and a pressure of between 0.2 and 0.6 MPa,
said impurities effluent feeding said step E).

9. Process according to Claim 8, in which said hydrated butadiene effluent resulting from said second gas-liquid separation section feeds a distillation section which separates from said hydrated butadiene effluent, at the bottom, an oxygenated compounds residue and, at the top, a hydrated butadiene effluent depleted in oxygenated compounds, said hydrated butadiene effluent depleted in oxygenated compounds then feeding step D) as hydrated butadiene effluent.

10. Process according to Claim 9, in which said oxygenated compounds residue feeds said effluent treatment step E) as a mixture with said contaminated reagents residue resulting from step C).

11. Process according to one of Claims 4 to 7, in which said hydrated butadiene effluent obtained at the end of said distillation section feeds a water-washing section comprising at least one gas-liquid washing section fed at the bottom with said hydrated butadiene effluent and at the top with a water stream, and producing, at the top, a hydrated butadiene effluent, depleted in oxygenated compounds, which feeds step D), and, at the bottom, a spent water raffinate.

12. Process according to Claim 8, in which said hydrated butadiene effluent obtained at the end of said second gas-liquid separation section feeds a water-washing section comprising at least one gas-liquid washing section fed at the bottom with said hydrated butadiene effluent and at the top with a water stream, and producing, at the top, a hydrated butadiene effluent, depleted in oxygenated compounds, which feeds step D), and, at the bottom, a spent water raffinate.

13. Process according to one of the preceding claims, in which said solvent of said 1-butene separation section is chosen from the group consisting of dimethylformamide (DMF), N-methylpyrrolidone and acetonitrile.

14. Process according to one of the preceding claims, in which the solvent residue resulting from the 1-butene separation section and the solvent residue resulting from the section for separating the oxygenated compounds are mixed before feeding the 1-butene separation section and the section for separating the oxygenated compounds, as a stream comprising a solvent.

15. Process according to one of the preceding claims, in which said step E) comprises at least a washing/backwashing section, a section for distilling light brown oils, a section for distilling heavy brown oils, an acetaldehyde separation section and an ethanol-water separation section.
